# EUROPEAN PATENT SPECIFICATION

(11) **EP 2 052 069 B1**
(45) Date of publication and mention of the grant of the patent: **11.12.2013**
(21) Application number: 07763784.1
(22) Date of filing: 18.07.2007
(51) Int. Cl.: C11D 7/26, C11D 3/48, C11D 7/42, A61L 2/18, C11D 11/00, C11D 3/00, C11D 17/00, C11D 7/34, C11D 7/50, C11D 3/386

(54) **LOW FOAMING CLEANER**
SCHAUMARMES REINIGUNGSMITTEL
AGENT DE NETTOYAGE À FAIBLE MOUSSAGE

(30) Priority: 18.07.2006 AU 2006903863 P; 07.02.2007 AU 2007900582 P
(43) Date of publication of application: 29.04.2009
(73) Proprietor: NOVAPHARM RESEARCH (AUSTRALIA) PTY. LIMITED, Rosebery, NSW 2018 (AU)
(72) Inventor: KRITZLER, Steven, Cronulla, NSW 2230 (AU); SAVA, Alex, Paddington, NSW 2021 (AU)
(74) Representative: Grünecker, Kinkeldey, Stockmair & Schwanhäusser
(86) International application number: PCT/AU2007/000999
(87) International publication number: WO 2008/009053

(56) References cited:
- EP-A1- 1 081 215
- GB-A- 1 286 054
- GB-A- 1 590 445
- US-A- 5 082 585
- US-A- 6 083 897

## Description

### Field of the invention:

This invention relates to a composition for use for general cleaning, and in particular for use in cleaning medical instruments and which is effective for soil removal and protein digestion while remaining low foaming.

### Background

The incidence has been widely reported of post procedural infections associated with surgery or diagnostic studies. It is believed that a significant number of these infections are due to inadequate reusable instrument reprocessing.

Cleaning of instruments on an industrial scale involves two steps. In the first step the instrument is cleaned and in the second step it is disinfected normally to "high level disinfection" or "sterilization" standards. It is generally accepted that failure to adequately clean items after use in the first step may compromise the efficacy of the second. The elimination of human proteins from the instruments represents a significant challenge. The challenge has been made more difficult as medical instruments have been developed, for example endoscopes, which utilize materials that are neither temperature resistant nor chemically inert.

For effective cleaning of medical instruments a preparation should be effective for soil removal, effective for protein digestion and resist foaming. In addition, the products are required to have stability and a long shelf life.

These desiderata tend to be mutually inconsistent objectives. In order to avoid foaming, soil removal preparations used in hospital cleaning/sterilizing "reprocessing" systems have mainly utilized highly alkaline non-foaming detergents, but their use is incompatible with both enzymes, and with materials of construction of flexible endoscopes. The use of close to neutral "enzymatic detergents" (preparations including both enzymes and detergents) has been found to be relatively effective for removal of proteins and safe with endoscopes, and enables acceptable levels of soil removal to be achieved. However, while enzymes in "enzymatic detergents" help to remove proteins, surfactants have been needed to remove the fats and carbohydrates. Due to the incorporation of surfactants, "enzymatic detergents" tend to produce foam to an unacceptable extent.

Foaming is undesirable because it blocks the visualization of instruments in manual cleaning baths, impedes access of washing liquor to soils during manual cleaning and blocks water jets and washing liquor circulation in automated washers (e.g. tunnel washers). The foams tend to block the lumens of instruments preventing effective cleaning of the lumen interior. When enzyme based cleaners have been used in reprocessing machinery the foam tends to fill the volume thus impeding the cleaning cycle by disrupting jets and agitation. Furthermore it makes the machine difficult to unload, interfering with proper draining, and leaving foam residues containing pathogens which can contaminate following cleaning cycles giving rise to significant risk of cross infection since the cleaners do not kill the microorganisms which they dislodge from surfaces. Instruments covered with foam require additional handling and washing before they can be sterilized. Increasingly the additional labour cost, time, and water consumption costs are regarded as unacceptable. Multiple guidelines and standards recognise the problem and warn against using foaming detergents for cleaning medical instruments (e.g. AS 4187:2003 or AS 4815:2006).

Although this problem has been recognized, it has not to date been satisfactorily overcome. Two solutions to the foaming problem have been utilized, however to date neither approach has succeeded in satisfying the market need.

In the first approach antifoams have been added to the cleaning composition or washer, but that has been unsatisfactory because antifoams leave unacceptable residues on the medical instruments. In the second approach attempts have been made to use so called "low foaming" non-ionic detergents such as alkylene oxide adducts. These tend to leave an undesirable film of oily residue on treated surfaces similar to that from antifoams and also produce hazy solutions which reduce visibility during washing cycles.

As a consequence commercially available formulations results tend to be either inadequately cleansing, or high foaming, and thus not suitable for use for cleaning medical instruments, or tend to be unstable and possess an inadequate shelf life, due to denaturing of the enzymes by surfactants employed.

Cheetham (Australian Infection Control, Sept 2005, 10, 3, p103-109) compared 17 market leading enzyme based medical instrument cleaners from eight manufacturers (Table 1).

**Table 1 Products compared by Cheetham**

| (Australian Infection Control, Sept 2005, 10, 3, p103-109) | |
|---|---|
| PRODUCT | SUPPLIER/MFR |
| Cidezyme/Enxol | Johnson & Johnson |
| Endozyme | Ruhof |
| Endozyme AW plus | Ruhof |
| 3E-zyme/Omni-Zyme | Medisafe |
| Lapcholyzime | Ruhof |
| 3M Rapid Multi-Enzyme Cleaner 70500 | 3M |
| 3M Rapid Multi-Enzyme Cleaner 70501 | 3M |
| 3M Rapid Auto Multi-Enzyme Cleaner 70505 | 3M |
| Matrix | Whiteley Med. |
| Mediclean | Neodisher |
| Mediclean Forte | Neodisher |
| Medizym | Dr Weigert |
| Medizyme | Whiteley Med. |
| Mucadont Zymaktiv | Merz |
| Mucapur ER | Dr Welger |
| Orthozime | Ruhof |
| Pacer Release | Campbell Bros. |
| Prepzyme | Ruhof |

The products were tested using SDS - PAGE methodology to compare the molecular weights of a group of standardised blood proteins before and after exposure to the various cleaning products. Cheetham reported that half of the products tested, when used in accordance with the manufacturers' directions, exhibited little or no protein digestion, and only two of the products (Rapid 70500 and Rapid 70501 - both from 3M and also known as RMEC 70500 and RMEC 70501 respectively) provided a high degree of protein digestion. Cheetham did not report on foaming properties or stability. The present Applicant has tested the two products which provided a high degree of protein digestion and found that one exhibits high level of foaming while the other contains alkylene oxide block copolymer and leaves undesirable oily residues on the treated surface. Moreover, while both exhibit good stability with easily inhibited enzymes, both show poor stability with difficult to inhibit enzymes.

Further, whilst the problem has been outlined with respect to cleaning medical instruments, the desire for cleaning compositions which are efficacious in removing.soil and digesting proteins whilst resisting foaming is not limited to the field of cleaning medical instruments. Such properties, along with stability and a long shelf life, are desirable in many different cleaning applications.

A further area where low foaming cleaning compositions are desirable is in the area of air conditioning and cooling. For instance, fresh food cool rooms have their temperature controlled by a refrigeration unit fitted with fans which is integral with the room. The fans draw environmental air through a refrigerated cooling coil heat exchanger into the room. The process of cooling the air results in a lowering of humidity with the moisture being condensed onto the cold surfaces of the heat exchanger. It is well known that any environmental surface which is continually wet or damp will become covered in biofilm. This biofilm not only reduces heat exchange efficiency, but is a very significant potential source of microbiological contamination into the room and is therefore undesirable.

There currently only limited number of existing methods of removing biofilm from heat exchange coils. The biofilm may be removed with abrasive brushes or high pressure water. This has proved to be problematic because the spaces between the cooling fins are insufficient to allow efficient brushing and the surface areas so extensive as to make this brushing an extremely tedious process. High pressure water has proven to be undesirable because it damages the cooling fins which are made of thin aluminium sections.

Alternatively, the heat exchange coil may be washed with strong alkali or strong acid. This has proved to be problematic because the alkali or acid, whilst eventually removing the biofilm both causes significant corrosive damage to the aluminium fins and the copper refrigeration tubes to which they are attached. This corrosion severely limits the service life of the heat exchange coil.

EP1081215A1 describes a liquid enzymatic cleaning agent concentrate comprising protease, 18% sodium cumene sulfonate and 5% by weight of monooctyl ether of tetraethylene glycol as essential components. Additionally, surfactants, su ch as ethylene or propylene oxide with fatty alcohols containing 8 to 14 carbon atoms are required in order to promote removal of fats and carbohydrates.

Thus, it is desirable to have effective yet non-corrosive cleaning agents that act without producing large quantities of foam.

Any discussion of the prior art throughout the specification should in no way be considered as an admission that such prior art is widely known or forms part of common general knowledge in the field.

### Object of the invention

It is an object of the present invention to provide an improved composition for cleaning, and in particular cleaning medical instruments which avoids or ameliorates at least some of the disadvantages of prior art. It is an object of preferred embodiments of the present invention to provide a composition for cleaning, and in particular cleaning medical instruments which is low foaming, has excellent enzyme shelf stability and is effective for soil removal and protein digestion.

### Brief Statement of invention.

The present invention provides liquid compositions which provide high levels of soil removal, exhibit superior protease stability, and minimize foaming to acceptable levels without leaving undesirable levels of residues. The compositions exhibit very high enzyme shelf life stability.

In a broad aspect, the invention provides a liquid for cleaning, said composition excluding surfactants and comprising one or more enzymes including a protease, a solvent system including a water soluble glycol ether solvent, at least one anionic hydrotrope, and wherein the molar ratio of said at least one hydrotrope to said glycol ether in the composition is selected to preserve the activity of said one or more enzymes, as defined in claim 1.

According to a first aspect the invention provides a liquid composition for cleaning medical instruments, said composition excluding surfactants and comprising one or more enzymes including a protease, a solvent system including a water soluble glycol ether solvent, at least one anionic hydrotrope, and wherein the molar ratio of said at least one hydrotrope to said glycol ether in the composition is selected to preserve the activity of said one or more enzymes, as defined in claim 1.

Unless the context clearly requires otherwise, throughout the description and the claims, the words "comprise", "comprising", and the like are to be construed in an inclusive sense as opposed to an exclusive or exhaustive sense; that is to say, in the sense of "including, but not limited to".

In preferred embodiments the composition includes several additional hydrolase enzymes in addition to a protease or proteases, said hydrolase enzymes including but not limited to lipases, cellulases and amylases.

Desirably, the hydrotrope is an anionic hydrotrope selected from the group consisting of water soluble anionic hydrotropes of the formula: 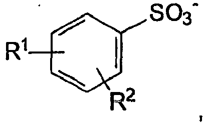 and more preferably of the formula 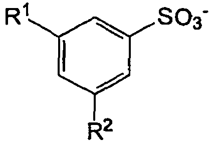 and having no alkyl side chain greater than six carbons in length.

In preferred hydrotropes R¹ and R² are independently alkyl groups of from 1 to six carbons, although R¹ or R² may optionally be hydrogen. Preferred hydrotropes have a short chain (less than six, and preferably from one to four carbons, and more preferably from one to two carbons). Very highly preferred hydrotropes are water soluble xylene sulfonate (R¹ is methyl, R² is methyl) and cumene sulfonate (R¹ is isopropyl, R² is hydrogen) salts.

Since both anionic hydrotropes and glycol ether solvents are considered strong protein (and enzyme) denaturing agents it is surprising that compositions according to the invention possess all the above desiderata:
- non foaming
- excellent enzyme shelf-life stability
- excellent cleaning performance against standard medical soils,
- leaves no undesirable residues

According to the invention the molar ratio of hydrotope: glycol ether is selected to be 1.1:1 or greater. More preferably the weight ratio of hydrotope: glycol ether is greater than 1.2: 1 or better still is greater than 1.5:1.

According to a further aspect the invention provides a composition according to the first aspect in a concentrate adapted to be diluted for use by at least 20 parts of water to 1 part of the concentrate (100 to 1000 parts of water to 1 part of concentrate in preferred embodiments) and wherein the hydrotrope is selected from the group comprising of water soluble aromatic sulfonates with one or more short (C1-C6) side alkyl chains..

According to a further aspect the invention provides a composition according to the first aspect wherein the solvent comprises in combination at least one glycol ether, at least one polyhydric alcohol, and water containing boron or borate ions.

According to a further aspect the invention provides a composition according to any one of the preceding aspects wherein each component of the composition is selected so as to exclude compounds incorporating an alkyl chain of longer than six carbons.

The concentration ratios are critical for prevention of enzyme deterioration on storage. The weight ratio of hydrotrope to proteolytic enzyme to should be between 400:1 and 200:1, more preferably 300:1 and 350:1 and the concentration of hydrotrope should not exceed 25 %. The molar ratio of glycol ether to polyhydric alcohols is preferably between 0.2:1 and 1:1

The compositions of the present invention are particularly suited to cleaning medical instruments, and have been principally described with reference to that use, however, it will be appreciated that the cleaning compositions of the present invention are by no means limited to that use. They may be used in any circumstances where it is desired to clean biological matter from surfaces, including industrial and domestic applications, for example, in cleaning down any wet surface contaminated with proteinaceous materials, or cleaning refrigeration coils. The compositions of the present invention have been found to be especially efficacious for cleaning the interior of cooling towers and the heat exchange surfaces of heat exchange equipment involving water.

### EXAMPLES:

Compositions according to the invention are shown in examples 1, 2, 3.
These differ from each other primarily in that the molar ratio of sodium xylene sulfonate to glycol ether in the compositions is 1.1:1; 1.2:1 and 1.6:1 respectively.

### Example 1 (molar ratio hydrotrope to glycol ether 1.1:1)

| Component | Preferred %w/w |
|---|---|
| Sodium xylene sulfonate | 13.8 |
| proteolytic enzyme | 0.06 |
| Selected other enzymes | 0.02 |
| glycerol | 4.1 |
| Propylene glycol | 12 |
| glycol ether | 8.9 |
| Preservative | 0.1 |
| Borax | 4.1 |
| 5% Calcium solution | 0.5 |
| water | balance |

### Example 2 (molar ratio hydrotrope to glycol ether 1.2:1

| Component | Preferred %w/w |
|---|---|
| Sodium xylene sulfonate | 16 |
| protease | 0.09 |
| Selected other enzymes | 0.01 |
| glycerol | 5 |
| Propylene glycol | 4 |
| glycol ether | 9.5 |
| Preservative | 0.1 |
| Borax | 2 |
| 5% Calcium solution | 0.5 |
| water | balance |

### Example 3 (molar ratio hydrotrope to glycol ether 1.6:1

| Component | Preferred %w/w |
|---|---|
| Sodium xylene sulfonate | 15 |
| Protease | 0.05 |
| Selected other enzymes | 0.02 |
| glycerol | 6 |
| Propylene glycol | 5 |
| glycol ether | 6.6 |
| Preservative | 0.1 |
| Borax | 3 |
| 5% Calcium solution | 0.1 |

Comparative examples 4, 5 are similar to example 1 except that the mole ratio of hydrotrope to glycol ether is 1.0:1.0 in example 4; and is 0.9:1 in example 5

### Comparative examples 4 and 5

| | Comparative Example 4 | Comparative Example 5 |
|---|---|---|
| Ratio of hydrotrope to glycol ether | 1.0:1 | 0.9:1 |
| Sodium xylene sulfonate | 18 | 15 |
| Protease | 0.07 | 0.09 |
| Selected other enzymes | 0.02 | 0.02 |
| Glycerol | 3.6 | 5 |
| Propylene glycol | 4 | 4 |
| glycol ether | 12.7 | 11.7 |
| Preservative | 0.8 | 0.8 |
| Borax | 2 | 6 |
| 5% Calcium solution | 0.5 | 0.5 |

In use, compositions according to the invention may be stored as concentrates for periods of at least 18 months at 25°C and should be diluted by tap water from 20:1 to 1000:1 before use.

Table 2 below summarises the performance of the best of the compositions evaluated by Cheetham as referred to above and identified in table 1. Table 2 compares in summary form 12 commercially available cleaners in terms of shelf life protease stability (columns 2 and 3), soil removal efficacy (column 4), residual foam height (column 5) and presence of potential residue. The three most effective commercially available compositions in terms of soil removal were Cidezyme, 3M Rapid 70505 and 3M 70500 all of which scored 10. However, of these 3M Rapid 70500 produced a residual foam height of 500 ml which is unacceptable, while 3M rapid 70505 left an oily residue which is also unsatisfactory. The Cidezyme passed the residual foam height test without any residue. However Cidezyme failed on both the stable and unstable proteases shelf life stability tests. In comparison formulations according to examples 1, 2, 3 of the invention achieved excellent soil removal and passed each of the tests.

**Table 2**

| Enzymatic detergent | Stable protease shelf life (Test A) | Unstable protease shelf life (Test B) | Soil Removal Test (10= best; 0= worst) | Foam volume Test, ml at 25C, | Residue Presence test |
|---|---|---|---|---|---|
| Dr2000 NT-1 | **fail** | **fail** | 7 | **fail** | |
| Orthozyme | **fail** | **fail** | 3 | **fail** | |
| Pacer Release | **fail** | **fail** | 7 | **fail** | |
| Omnizyme | **fail** | **fail** | 6 | pass | pass |
| Medizyme | nt | nt | 6 | fail | |
| Lapcholyzime | nt | nt | 5 | pass | pass |
| Endokleen | **fail** | **fail** | 4 | **fail** | |
| Endozyme | nt | **fail** | 6 | **fail** | |
| Endozyme AW plus | **fail** | **fail** | 6 | **fail** | |
| **Cidezyme** | **fail** | **fail** | 10 | pass | pass |
| **3M Rapid Auto 70505** | pass | **fail** | 10 | pass | **fail** |
| **3M Rapid Auto 70500** | pass | **fail** | 10 | **fail** | pass |
| **Invention Example 1** | **pass** | **pass** | **10** | **pass** | **pass** |
| **Invention Example 2** | **pass** | **pass** | **10** | **pass** | **pass** |
| **Invention Example 3** | **pass** | **pass** | **10** | **pass** | **pass** |

Table 3 below shows the results for comparative examples 4 and 5. These examples differ from examples 1 to 3 in that the molar ratio of hydrotrope to glycol ether is not selected to preserve the activity of said one or more enzymes, and is below 1.0:1 and 0.9:1 respectively. This shows that to achieve stability for the compositions exemplified the mole ratio of hydrotrope to glycol ether should be selected to be above 1.1:1. However the ratio required to be selected could be determined for other compositions within the scope of the invention having regard to the teachings herein disclosed.

**Table 3**

| Enzymatic detergent | Protease stability Test A | Protease Stability Test B | Soil Removal Test (10= best; 0= worst) | Residual Foam Test | Residue test |
|---|---|---|---|---|---|
| Comparative example 4 | **fail** | **fail** | 10 | pass | Pass |
| Comparative example 5 | **fail** | **fail** | 10 | pass | pass |

Details of the tests used and results obtained to prepare the data in tables 2 and 3 above are given below:

### 1. Soil removal test

**Scope:** This method allows for a qualitative and/or quantitative assessment of the relative efficacy of cleaners and detergents in removing a simulated medical soil.

Browne indicator strips - STF load check indicators (Albert Browne Ltd Leicester UK) - are designed to ensure and assist in documenting the cleaning efficacy of tunnel washers, single chamber washer-disinfectors, etc. The indicator consists of a plastic substrate, with a patch of protein-based soil applied to both sides. This simulates a very difficult to remove medical soil. The amount of soil remaining on the strip after detergent treatment can be assessed visually.

### Preparation of samples for Soil removal test

125 ml beakers with 99±0.5 ml of tap water are placed in a water bath to equilibrate to required temperature for approximately 30 minutes.
The required amount of test product/sample detergent is then added to each beaker and stirred gently. One beaker is left as a control with the addition of 1ml of water instead of test product. These solutions are left for a further 5 minutes to equilibrate to temperature.

Browne STF Load Check Indicator strips (Browne strip) are cut in half (to give two test strips) and then added to each beaker. The dimensions of the beaker are selected to enable the strip to be positioned at an angle whilst being fully submerged in the test solution.

At the end of the prescribed time interval the strips are carefully removed with clean tweezers ensuring that no contact is made with the soiled patch on either side of the strip. The strips were then dipped in clean tap water briefly and then allowed to drip dry. After drying the strips are placed on white paper and photographed for visual assessment.

### Estimation of the degree of soil removal

The degree of soil removal is generally measured on a scale of 0 to 10, with 0 being the lowest degree (No visible soil removal) and 10 being the highest degree (complete soil removal).

### (b) Soil removal Results

The best commercially available enzymatic detergents (per Cheetham - see appended table 1) were compared with formulations according to the invention using the soil removal test described above with the results shown in Table 4.

**Table 4**

| Enzymatic detergent | Removal Test (10= best; 0= worst) |
|---|---|
| Dr2000 NT-1 | 7 |
| Orthozyme | 3 |
| Pacer Release | 7 |
| Omnizyme | 6 |
| Medizyme | 6 |
| Lapcholyzime | 5 |
| Endokleen | 4 |
| Endozyme | 6 |
| Endozyme AW plus | 6 |
| **Cidezyme** | 10 |
| **3M Rapid Auto 70505** | 10 |
| **3M Rapid Auto 70500** | 10 |
| **Invention Example 1** | **10** |
| **Invention Example 2** | **10** |
| **Invention Example 3** | **10** |
| Comparative example 4 | 10 |
| Comparative example 5 | 10 |

### 2. Protease Shelf Life stability Tests

**Scope:** The test allows comparison of ingredients of enzymatic formulations in respect of their ability to preserve protease activity during storage. Enzymatic activity is known to decrease over time due to protein denaturing and auto-proteolysis (self-digestion). These processes are dramatically accelerated by increase in temperature - each 10 degrees temperature rise increases the rate of denaturing by up to 8 times. The loss of proteolytic activity over time is quantified for each product and expressed as percentage for each formulation.

### Procedure:

Denature any remaining protease in cleaners under study by gentle boiling of each product for 2-3 min in a capped beaker,
1. Cool and confirm absence of proteolytic activity using protease test strips,
2. Add 10% w/w of test protease. In "Test A" a stable protease (Savinase Ultra 16XL, from Novozymes) is used. In "Test B" a relatively unstable enzyme (Savinase 16L, from Novazymes) is used. If practical, both the well stabilised and a poorly stabilised enzyme are used in the same assay - e.g. Savinase Ultra 16XL AND Savinase 16L from Novozymes.
3. Divide each prepared sample into three and store at 4, 25 and 40 °C
4. Assay and report initial protease activity
5. After 14 days assay remaining protease activity of each sample Report the percentage of protease activity loss at each temperature.

A loss of 5% or less of initial protease activity for both stable and unstable proteases in table 5 is regarded as a "pass".

### (b) Results for stable and unstable protease shelf life tests.

The results obtained for each of the compositions listed in table 4 in respect of stable and unstable Protease shelf life tests described above is shown in Table 5:

**Table 5**

| Enzymatic detergent | Stable protease shelf life Test A | Unstable protease shelf life Test B |
|---|---|---|
| Dr2000 NT-1 | **29.1** | **51** |
| Orthozyme | **>35** | **>50** |
| Pacer Release | **>35** | **>50** |
| Omnizyme | **>35** | **>50** |
| Medizyme | nt | nt |
| Lapcholyzime | nt | nt |
| Endokleen | **>35** | **>50** |
| Endozyme | nt | **>50** |
| Endozyme AW plus | **>35** | >50 |
| **Cidezyme** | **21.1** | **38.9** |
| **3M Rapid Auto 70505** | <5 | **11.5** |
| **3M Rapid Auto 70500** | <5 | **12.5** |
| Invention Example 1 | <5 | <5 |
| Invention Example 2 | <5 | <5 |
| Invention Example 3 | <5 | <5 |
| Comparative example 4 | 22.4 | 12.1 |
| Comparative example 5 | 19.5 | 16.1 |
| Comparative example 6 | 6 | 11.9 |

| | | |
|---|---|---|
| nt=not tested | | |

### 3 Foam volume test and Residue Presence tests

### Principle (foam volume)

An increase in foam volume was determined by blending for 30 secs using a commercial type blender with glass jar at 25 ± 1 °C agitated at ∼ 6000 rpm, and then measuring the increase in total volume of test fluid including foam.

### Apparatus

Blender: A Moulinex commercial blender was used. The glass jar was volume graduated (20-25mL marks).

### Procedure (foam volume)

1. Clean and rinse the blender with distilled water using 10s blends and fresh samples of distilled water until blending develops no appreciable foam. If a foam persist, clean with alcohol, followed by at least three rinses with distilled water.
2. Using the manufacturer's recommended dilutions prepare 500 ml of solution
3. Pour the test liquid into a clean glass bottle or jar and store it at 25°± 1°C for a minimum of 1 h and a maximum of 2 h in the constant temperature water bath deep enough so that the water level is at least 10 mm above the air/test fluid interface.
4. Pour the test liquid into the blender jar.
5. Measure and record the test liquid volume, disregarding any foam. Call this the initial volume I.
6. Blend for 30 ± 1 s at selected speed.
7. Shut off the blender and immediately measure the total volume including foam. Subtract initial volume of solution (I) and report as foam volume.

A residual foam height of less than 100 is accepted as a "pass".

### Residue Presence Test

**Scope:** Report oily residues, if present.
**Method:** Oily residues can be easily observed on glass slides using dissecting microscope and lateral lighting.

Pre cleaned microscope glass slides were dipped into diluted enzymatic cleaner and then gently rinsed by dipping the slide once into a beaker with distilled water. The slide was allowed to drip dry before assaying for presence of residues

Any detectable residue is a "fail". No detected residue is a "pass".

### (b) Results for Residual Foam Volume and Residue Presence tests.

The results obtained for each of the compositions listed in table 4 in respect of the foam volume test and residue presence test described above is shown in Table 6:

**Table 6**

| Enzymatic detergent | Residual Foam Test | Residue detection test |
|---|---|---|
| Dr2000 NT-1 | **>500** | **fail** |
| Orthozyme | **100** | pass |
| Pacer Release | **350** | pass |
| Omnizyme | <25 | pass |
| Medizyme | 150 | **fail** |
| Lapcholyzime | <25 | pass |
| Endokleen | **200** | **fail** |
| Endozyme | **175** | |
| Endozyme AW plus | **200** | |
| **Cidezyme** | <25 | pass |
| **3M Rapid Auto 70505** | <25 | **fail** |
| **3M Rapid Auto 70500** | **>500** | pass |
| Invention Example 1 | <25 | pass |
| Invention Example 2 | <25 | pass |
| Invention Example 3 | <25 | pass |
| Comparative example 4 | <25 | pass |
| Comparative example 5 | <25 | pass |
| Comparative example 6 | <25 | pass |

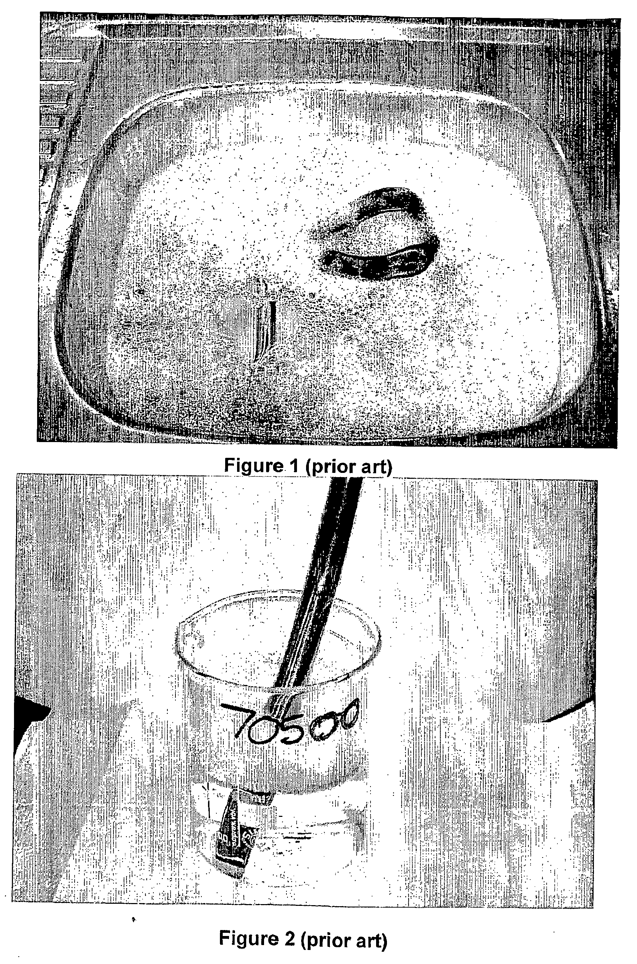

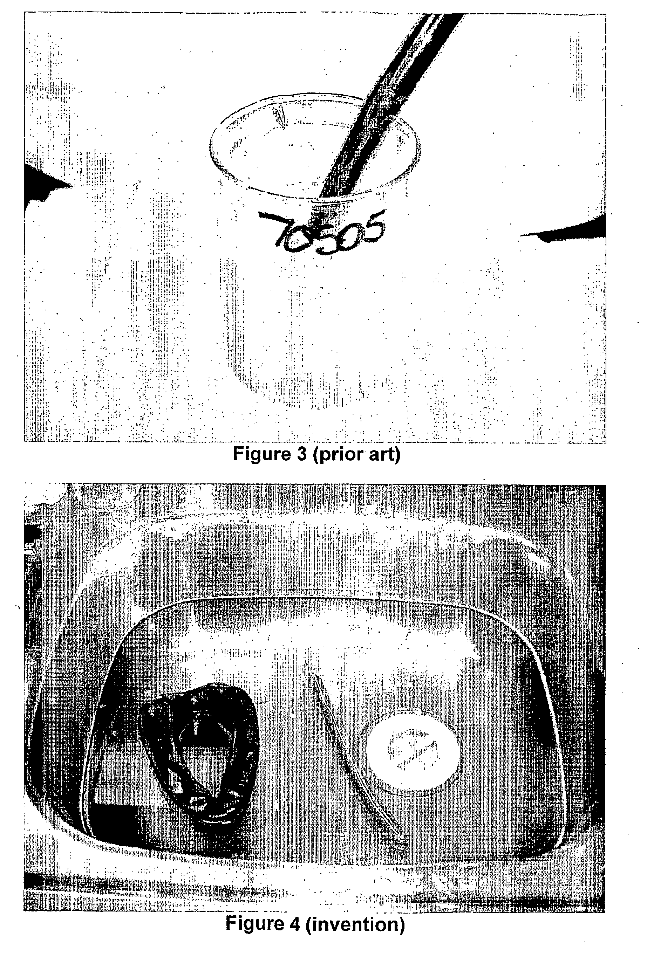

By way of further example, appended figures 1 - 4 illustrate differences in foaming/residue properties. Figures 1 - 4 simulate normal usage procedures in which a concentrate is measured into a container and then the required amount of water is added. The result is photographed without stirring.
Figure 1 shows medical instruments in a container filled with 3M Rapid Multi enzyme Cleaner 70500 - one of the two best performers in the Cheetham study. The instruments are hardly visible because of foam.
Figure 2 shows the same product (3M Rapid Multi enzyme Cleaner 70500) in a beaker with a stable volume of foam above the liquid.
Figure 3 shows the other of the best performers (3M Rapid 70505). A visible undesirable milky residue is suspended in the cloudy liquid.
Figure 4 corresponds to figure 1 when a composition according to the invention (example 2) is employed.

In the compositions exemplified the ratios of hydrotrope to protease and of DPM to polyhydric alcohols for each of the compositions is shown in table 7

**Table 7**

| Composition | Ratio Hydrolase to protease | Ratio DPM to polyhydric alcohols |
|---|---|---|
| Example 1 | 230:1 | 0.3 |
| Example 2 | 177:1 | 0.6 |
| Example 3 | 300:1 | 0.34 |
| Comparative Example 4 | 257:1 | 0.93 |
| Comparative Example 5 | 166:1 | 0.73 |

### Example 7 - Cleaning heat exchanger

The low foaming compositions of the present invention was used to clean a heat exchanger. A two step process was employed.

Firstly the heat exchanger was sprayed with the enzymatic cleaner of the present invention such as described in Examples 1-3 above. The enzymatic cleaner is typically diluted at a rate of 50 parts water to 1 part enzymatic cleaner for very dirty heat exchangers and up to 100 parts of water to 1 part of enzymatic cleaner for less severely soiled heat exchangers.

The cleaner is allowed to soak into the contaminated surface in order to penetrate and digest biological matter. The soaking period is typically between 10 and 20 minutes depending on the depth of soil on the heat exchange surfaces.

Secondly, the heat exchanger was sprayed with low pressure water to remove the digested contaminants without physical damage to the fins. The digested contaminants were readily removed as the amount of foam obstruction of the coils was minimal.

This process was in contrast to carrying out the clearing with a conventional enzymatic preparations which have a propensity to foam copiously during this spraying phase. The foam suspends contaminant particles and hides from view the areas which require further spraying.

Therefore the use of a very low foaming or non foaming enzymatic preparation has proved to be greatly advantageous.

## Claims

1. A liquid composition for cleaning, said composition excluding surfactants and comprising one or more enzymes including a protease, a solvent system including a water soluble glycol ether solvent, at least one anionic hydrotrope, and wherein the molar ratio of said hydrotrope to said glycol ether in the composition is selected to be 1.1:1 or greater and wherein each component of the composition is selected so as to exclude compounds incorporating an alkyl chain of longer than six carbons.

2. A liquid composition according to claim 1 wherein the composition includes several additionall hydrolase enzymes in addition to a protease or proteases, said hydrolase enzymes including but not limited to lipases, cellulases and amylases.

3. A liquid composition according to claim 1 or claim 2 wherein the hydrotope is an anionic hydrotope selected from the group consisting of water soluble anionic hydrotopes of the formula: 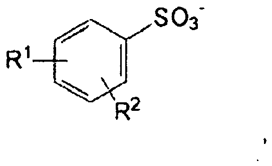 preferably 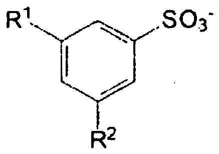 and having no alkyl side chain greater than six carbons in length.

4. A liquid composition according to any one of the preceding claims
wherein R¹ and R² are independently alkyl groups of from one to six carbons, although R¹ or R² may optionally be hydrogen.

5. A liquid composition according to any one of the preceding claims
wherein R¹ and R² have a chain from one to four carbons, preferably from one to two carbons.

6. A liquid composition according to any one of the preceding claims
wherein the hydrotope is xylene sulfonate or cumene sulfonate salts.

7. A liquid composition according to any one of the preceding claims
wherein the molar ratio of the hydrotrope: glycol ether is selected to be greater than 1.2:1 and preferably greater than 1.5:1.

8. A liquid composition according to any one of the preceding claims in a concentrate adapted to be diluted for use by at least 20 parts of water to one part of the concentrate (100 to 1000 parts of water to one part of concentrate in preferred embodiments) and wherein the hydrotope is selected from the group comprising of water soluble aromatic sulfonates with one or more short (C1-C6) side alkyl chains.

9. A liquid composition according to any one of the preceding claims wherein the solvent comprises in combination at least one glycol ether, at least one polyhydric alcohol, and water containing boron or borate ions.

10. A liquid composition according to any one of the preceding claims wherein the concentration of hydrotope does not exceed 25%.

11. A liquid composition according to any one of the preceding claims wherein the molar ratio of glycol ether to polyhydric alcohols is between 0.2:1 and 1:1.

12. A liquid composition according to any one of the preceding claims formulated for the use as a medical cleaner, industrial cleaner or for use in cleaning refrigerant coils.

13. A method of cleaning an device which is a medical instrument in need thereof or an airconditioning component in need thereof comprising the step of contacting said device with a composition according to any one of claims 1 to 12.

## Patentansprüche

1. Flüssige Zusammensetzung zur Reinigung, wobei die Zusammensetzung Tenside ausschließt und ein oder mehrere Enzyme einschießlich einer Protease, ein Lösungsmittel einschließlich eines wasserlöslichen Glykoletherlösungsmittels und mindestens ein anionisches Hydrotrop umfasst, und wobei das Molverhältnis des besagten Hydrotrops zu dem besagten Glykolether in der Zusammensetzung als 1,1:1 oder größer ausgewählt ist und wobei jede Komponente der Zusammensetzung so ausgewählt ist, dass sie Verbindungen ausschließt, die eine Alkylkette enthalten, die länger als sechs Kohlenstoffatome ist.

2. Eine flüssige Zusammensetzung nach Anspruch 1, wobei die Zusammensetzung mehrere zusätzliche Hydrolaseenzyme zusätzlich zu einer Protease oder Proteasen enthält, wobei die Hydrolaseenzyme Lipasen, Cellulasen und Amylasen beinhalten, jedoch nicht darauf beschränkt sind.

3. Eine flüssige Zusammensetzung nach Anspruch 1 oder Anspruch 2, wobei das Hydrotrop ein anionisches Hydrotrop ist, ausgewählt aus der Gruppe, bestehend aus wasserlöslichen anionischen Hydrotropen der Formel: 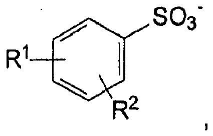 vorzugsweise 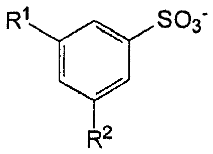 und ohne Alkyl-Seitenkette mit mehr als sechs Kohlenstoffatomen in der Länge.

4. Eine flüssige Zusammensetzung nach einem der vorhergehenden Ansprüche, wobei R¹ und R² unabhängig voneinander Alkylgruppen mit einem bis sechs Kohlenstoffatomen sind, obwohl R¹ oder R² optional Wasserstoff sein können.

5. Eine flüssige Zusammensetzung nach einem der vorhergehenden Ansprüche, wobei R¹ und R² eine Kette von einem bis vier Kohlenstoffatomen haben, vorzugsweise von einem bis zwei Kohlenstoffatomen.

6. Eine flüssige Zusammensetzung nach einem der vorhergehenden Ansprüche, wobei das Hydrotrop ein Xylolsulfonat- oder Cumolsulfonat-Salz ist.

7. Eine flüssige Zusammensetzung nach einem der vorhergehenden Ansprüche, wobei das Molverhältnis von Hydrotrop zu Glykolether so ausgewählt ist, dass es größer als 1,2:1 und vorzugsweise größer als 1,5:1 ist.

8. Eine flüssige Zusammensetzung nach einem der vorhergehenden Ansprüche in einem Konzentrat, geeignet zur Verdünnung für die Verwendung mit mindestens 20 Teilen Wasser auf ein Teil des Konzentrats (100 bis 1000 Teile Wasser zu einem Teil Konzentrat in bevorzugten Ausführungsformen) und wobei das Hydrotrop ausgewählt ist aus der Gruppe, umfassend wasserlösliche aromatische Sulfonate mit einem oder mehreren kurzen (C1-C6)-Alkylseitenketten.

9. Eine flüssige Zusammensetzung nach einem der vorhergehenden Ansprüche, wobei das Lösungsmittel in Kombination mindestens einen Glykolether, mindestens einen mehrwertigen Alkohol und Wasser, enthaltend Bor oder Borat-Ionen, umfasst.

10. Eine flüssige Zusammensetzung nach einem der vorhergehenden Ansprüche, wobei die Konzentration von Hydrotrop nicht mehr als 25% beträgt.

11. Eine flüssige Zusammensetzung nach einem der vorhergehenden Ansprüche, wobei das Molverhältnis von Glycolether zu mehrwertigen Alkoholen zwischen 0,2:1 und 1:1 liegt.

12. Eine flüssige Zusammensetzung nach einem der vorhergehenden Ansprüche, formuliert zur Verwendung als medizinischer Reiniger, industrieller Reiniger oder zur Verwendung bei der Reinigung von Kühlschlangen.

13. Verfahren zum Reinigen eines Geräts, das ein medizinisches Instrument ist, das derartiges bedarf, oder das eine klimatisierende Komponente ist, die derartiges bedarf, umfassend den Schritt des Inkontaktbringens des Geräts mit einer Zusammensetzung nach einem der Ansprüche 1 bis 12.

## Revendications

1. Composition liquide de nettoyage, ladite composition excluant les tensioactifs et comprenant un ou plusieurs enzymes comportant un protéase, un système de solvant comprenant un solvant d'éther glycol soluble dans l'eau, au moins un hydrotrope anionique et où le rapport molaire dudit hydrotrope audit éther glycol dans la composition est sélectionné pour être 1,1:1 ou davantage et où chaque composant de la composition est sélectionné pour exclure les composés incorporant une chaîne alkyle de plus de six carbones.

2. Composition liquide selon la revendication 1, dans laquelle la composition comprend plusieurs enzymes hydrolases supplémentaires en plus d'une protéase ou de plusieurs protéases, lesdits enzymes hydrolases, comprenant mais sans s'y limiter, les lipases, les cellulases et les amylases.

3. Composition liquide selon la revendication 1 ou la revendication 2, où l'hydrotrope est un hydrotrope anionique sélectionné parmi le groupe consistant en hydrotropes anioniques solubles dans l'eau de la formule: 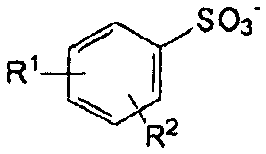 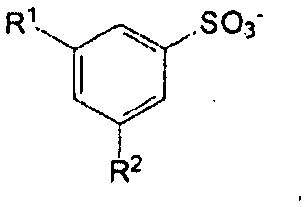 de préférence et ne comprenant aucune chaîne latérale alkyle de plus de six carbones de long

4. Composition liquide selon l'une quelconque des revendications précédentes
où R¹ et R² sont indépendamment des groupes alkyles d'un à six carbones, même si R¹ ou R² peut éventuellement être l'hydrogène.

5. Composition liquide selon l'une quelconque des revendications précédentes
où R¹ et R² possèdent une chaîne d'un à quatre carbones, de préférence d'un à deux carbones.

6. Composition liquide selon l'une quelconque des revendications précédentes ,
où l'hydrotrope est un sel de sulfonate de xylène ou un sel de sulfonate de cumène.

7. Composition liquide selon l'une quelconque des revendications précédentes ,
où le rapport molaire entre l'hydrotrope et l'éther glycol est sélectionné pour être supérieur à 1,2:1 et de préférence supérieure à 1,5:1.

8. Composition liquide selon l'une quelconque des revendications précédentes, dans un concentrée adapté pour une dilution d'utilisation à raison de 20 parties d'eau pour une partie du concentré (100 à 1000 parties d'eau pour une partie de concentré dans des modes de réalisation préférés) et où l'hydrotrope est sélectionné parmi le groupe comprenant les sulfonates aromatiques solubles dans l'eau avec une ou plusieurs courtes chaînes alkyles latérales (C1-C6).

9. Composition liquide selon l'une quelconque des revendications précédentes, où le solvant comprend en combinaison au moins un éther glycol, au moins un alcool polyhydrique et de l'eau contenant des ions de bore ou de borate.

10. Composition liquide selon l'une quelconque des revendications précédentes, où la concentration en hydrotrope n'excède pas 25%.

11. Composition liquide selon l'une quelconque des revendications précédentes, où le rapport molaire de l'éther glycol aux alcools polyhydriques est compris entre 0,2:1 et 1:1.

12. Composition liquide selon l'une quelconque des revendications précédentes formulée pour utilisation comme produit de nettoyage médical, produit de nettoyage industriel ou pour utilisation dans le nettoyage des bobines réfrigérantes.

13. Procédé de nettoyage d'un appareil, comme un instrument médical demandeur ou un composant de climatisation demandeur comprenant la phase consistant à mettre en contact ledit appareil avec une composition selon l'une quelconque des revendications 1 à 12.
